# EUROPEAN PATENT APPLICATION

(11) **EP 4 751 781 A2**
(43) Date of publication of application: **03.06.2026**
(21) Application number: 26155810.0
(22) Date of filing: 18.12.2020
(51) Int. Cl.: A61Q 11/00

(54) **ORAL CARE COMPOSITIONS AND METHODS OF USE**

(30) Priority: 20.12.2019 US 201962951592 P
(62) Divisional of application: 20842916.7
(71) Applicant: Colgate-Palmolive Company, New York, NY 10022 (US)
(72) Inventor: THOMSON, Paul, Piscataway, 08854 (US); D'AMBROGIO, Robert, Princeton, 08540 (US); DENIS, Jean, Union, 07083 (US); XU, Guofeng, Plainsboro, 08536 (US)
(74) Representative: Sonnenhauser, Thomas Martin

(57) **Abstract**

The invention relates to oral care compositions comprising a first stannous ion source, a second stannous ion source, wherein the second stannous ion comprises stannous pyrophosphate, and a source of zinc selected from zinc oxide, zinc citrate, zinc lactate, and combinations thereof, and a humectant system comprising glycerin and sorbitol, and a thickening system, as well as to methods of using and making these compositions.

## Description

### CROSS REFERENCE TO RELATED APPLICATIONS

This application claims the benefit and priority of United States provisional application 62/951592, filed on December 20, 2019, the contents of which are incorporated herein in their entirety.

### FIELD OF THE INVENTION

This invention relates to oral care compositions comprising a first stannous ion source, a second stannous ion source, wherein the second stannous ion comprises stannous pyrophosphate, and a source of zinc selected from zinc oxide, zinc citrate, zinc lactate, and combinations thereof, and a humectant system comprising glycerin and sorbitol, and a thickening system, as well as to methods of using and making these compositions.

### BACKGROUND

Oral care compositions present particular challenges in preventing microbial contamination.

Stannous ions, in particular stannous salts such as stannous fluoride, are known anti-microbial agents and are used in various dentifrices as agents for preventing plaque. However, there are certain disadvantages to using stannous salts, such as instability, tendency to stain teeth, astringency, and unpleasant taste for users.

Zinc is also a known antimicrobial agent used in toothpaste compositions. Zinc is a known essential mineral for human health, and has been reported to help strengthen dental enamel and to promote cell repair. Unfortunately, conventional toothpaste formulations often require high concentrations of zinc, e.g., 2% by weight or more, to achieve efficacy. At this concentration, the zinc imparts a notably astringent taste to the composition. There is thus a need for improved antibacterial toothpaste formulations that do not suffer from the drawbacks of conventional compositions.

However, due to formula complexity of certain oral care compositions which contain both stannous fluoride and one or more zinc sources, these formulas can be a challenge to produce from both a manufacturing and supply chain perspective.

Accordingly, in view of the drawbacks and disadvantages to using various antimicrobials, such as zinc and stannous, there is a need for oral care compositions with anti-bacterial efficacy, but which are also palatable and desirable for a user.

### BRIEF SUMMARY

Certain oral care compositions comprising stannous fluoride and zinc use glycerin as the main or primary humectant. However, it has been surprisingly found that, in those same zinc and stannous fluoride compositions, sorbitol can actually be used to supplement or partially replace glycerin. The addition of sorbitol, at certain percentages and weights, affords easier processing of the oral care composition but still allows the oral care formulations to maintain or even improve therapeutic performance in certain assays, as well as chemical and physical stability. Without being bound by theory, the addition of sorbitol at defined concentrations may allow for reduced heating and mixing time for processing for certain formula structuring agents, for example, hydroxyethylcellulose (HEC).

Furthermore, stannous fluoride formulations that contain zinc oxide and zinc citrate can provide much needed antibacterial benefits. However, another unexpected discovery is that stannous fluoride formulas with both sorbitol and glycerin, and which contain stannous pyrophosphate but only a single zinc source (i.e., zinc oxide), can provide enhanced antibacterial performance relative similar formulas that comprise both zinc oxide and zinc citrate.

For example, in one aspect the invention is an oral care composition (Composition 1.0) comprising:
- A zinc source selected from the group consisting of: zinc oxide, zinc citrate, zinc lactate, and combinations thereof (e.g., ZnO and ZnCit, or e.g., ZnO and ZnLac);
- a first source of stannous (e.g., stannous fluoride);
- a second source of stannous, wherein the second source of stannous comprises stannous pyrophosphate;
- a humectant system comprising glycerin and sorbitol, and wherein the sorbitol is in an amount from 2.5% - 35% by wt. of the total composition (e.g., about 3%, about 3.5%, about 5%, about 7%, about 10%, about 10.5%, about 14%, about 15%, about 20%, about 21%, about 24%, about 25%, about 30%) (e.g., 5% - 35% by wt of the total composition); and
- a thickening system comprising a thickening agent selected from the group consisting of carboxyvinyl polymers, carrageenan, hydroxyethyl cellulose, water-soluble salts of cellulose ethers (e.g., sodium carboxymethyl cellulose and sodium carboxymethyl hydroxyethyl cellulose), and combinations thereof.
For example, the invention contemplates any of the following compositions (unless otherwise indicated, values are given as percentage of the overall weight of the composition)
1.1 Composition 1.0, wherein the zinc source comprises zinc oxide.
1.2 Composition 1.0, wherein the zinc source comprises zinc oxide and zinc citrate (e.g., zinc trihydrate).
1.3 Any of the preceding compositions, wherein the ratio of the amount of zinc oxide (e.g., wt.%) to zinc citrate (e.g., wt%) is from 1.5:1 to 4.5:1 (e.g., 2:1, 2.5:1, 3:1, 3.5:1, or 4:1).
1.4 Any of the preceding compositions comprising zinc citrate and zinc oxide, wherein the zinc citrate is present in an amount of from 0.25 to 1.0 wt% (e.g., 0.5 wt. %) and zinc oxide may be present in an amount of from 0.75 to 1.25 wt% (e.g., 1.0 wt. %) based on the weight of the oral care composition.
1.5 Any of the preceding compositions wherein the zinc citrate (e.g., zinc trihydrate) is about 0.5 wt%.
1.6 Any of the preceding compositions wherein the zinc oxide is about 1.0 wt%.
1.7 Any of the preceding compositions where the zinc citrate (e.g., zinc trihydrate) is about 0.5 wt% and the zinc oxide is about 1.0 wt%.
1.8 Any of the preceding compositions, wherein the zinc oxide is about 1.0 wt%.
1.9 Any of the preceding compositions, where the zinc citrate is about 0.8 wt% (e.g., about 0.85 wt.%) and the zinc oxide is about 1.0 wt%.
1.10 Any of the preceding compositions, wherein the amount of stannous pyrophosphate is from 0.1% - 3% by wt. of the composition. (e.g., about 1% by wt. of the composition).
1.11 Any of the preceding composition, wherein the first stannous ion source is stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or a mixture thereof.
1.12 Composition of 1.11, wherein the first stannous ion source is stannous fluoride (e.g., about 0.45 wt%; e.g., about 0.454 wt%.)
1.13 Any of the preceding compositions further comprising a fluoride source selected from: stannous fluoride, sodium fluoride, potassium fluoride, sodium monofluorophosphate, sodium fluorosilicate, ammonium fluorosilicate, amine fluoride (e.g., N'-octadecyltrimethylendiamine-N,N,N'- tris(2-ethanol)-dihydrofluoride), ammonium fluoride, titanium fluoride, hexafluorosulfate, and combinations thereof.
1.14 Any of the preceding compositions wherein the pH is between 7.5 and 10.5.
1.15 Any of the preceding compositions further comprising an effective amount of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogen orthophosphate, monosodium phosphate, pentapotassium triphosphate and mixtures of any of two or more of these, e.g., in an amount of 1-20%, e.g., 2-8%, e.g., ca. 5%>, by weight of the composition.
1.16 The alkali phosphate salt of 1.15, wherein the salt comprises tetrapotassium pyrophosphate.
1.17 The composition of 1.16, wherein the tetrasodium pyrophosphate is from .1 - 3.0 wt% (e.g., about 2.0 wt%).
1.18 The composition of 1.17, wherein the salt comprises sodium tripolyphosphate.
1.19 The composition of 1.18, wherein sodium tripolyphosphate is from 0.1 - 3.0 wt% (e.g., about 2.0 wt%).
1.20 Any of the preceding compositions further comprising an abrasive or particulate (e.g., silica).
1.21 Any of the preceding compositions wherein the silica is synthetic amorphous silica. (e.g., 1% - 28% by wt.) (e.g., 8% - 25% by wt.).
1.22 Any of the preceding compositions wherein the silica abrasives are silica gels or precipitated amorphous silicas, e.g. silicas having an average particle size ranging from 2.5 microns to 12 microns.
1.23 Any of the preceding compositions further comprising a small particle silica having a median particle size (d50) of 1- 5 microns (e.g., 3 - 4 microns) (e.g., about 5 wt. % Sorbosil AC43 from PQ Corporation, Warrington, United Kingdom).
1.24 Any of the preceding compositions wherein 20-30 wt% of the total silica in the composition is small particle silica (e.g., having a median particle size (d50) of 3 -4 microns) and wherein the small particle silica is about 5 wt.% of the oral care composition.
1.25 Any of the preceding compositions comprising silica wherein the silica is used as a thickening agent, e.g., particle silica.
1.26 Any of the preceding compositions further comprising glycerin, wherein the glycerin is in a total amount of 30- 45% (e.g., about 42%).
1.27 The composition of 1.30, wherein the glycerin is in an amount of about 42% by wt. of the composition.
1.28 Any of the preceding compositions, wherein the composition comprises an aqueous buffer system, for example, wherein the buffer system comprises an organic acid and an alkali metal salt thereof, e.g., wherein the organic acid is citric acid and the salt is a mono-, di- and/or tri- alkali metal citrate salt, e.g., mono-, di- and/or tri- lithium, sodium, potassium, or cesium citrate salt, and citric acid.).
1.29 Composition of 1.28, wherein the buffer system comprises tri-sodium citrate and citric acid (e.g., 1 to 10% by weight of the composition) (e.g., 2.1% by wt. of the composition). For example, the molar ratio of mono-, di- and/or tri-sodium citrate and citric acid is 1.5 to 5, (e.g., 2 to 4).
1.30 Composition of 1.28 or 1.29, wherein the buffer is a citrate buffer comprising sodium citrate (e.g., about 1.5% wt.) and citric acid (e.g., about 0.6% wt.)
1.31 Any of the preceding compositions comprising polymer films.
1.32 Any of the preceding compositions comprising flavoring, fragrance and/or coloring.
1.33 Any of the preceding compositions wherein the thickening system comprises hydroxyethyl cellulose (e.g., hydroxyethyl cellulose and carrageenan) (e.g., from 0.2% - 0.75% total wt% of hydroxyethyl cellulose and carrageenan).
1.34 Any of the preceding compositions, wherein the thickening system comprises sodium carboxymethyl cellulose (e.g., from 0.5 wt.% - 1.5 wt.%).
1.35 Any of the preceding compositions comprising from 5% - 40%, e.g., 10% - 35%, e.g., about 10%, 15%, 25%, 30%, and 35% water.
1.36 Any of the preceding compositions comprising an additional antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, Zinc Chloride, Zinc Lactate, Zinc Sulfate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.37 Any of the preceding compositions comprising an antioxidant, e.g., selected from the group consisting of Co-enzyme Q10, PQQ, Vitamin C, Vitamin E, Vitamin A, BHT, anethole-dithiothione, and mixtures thereof.
1.38 Any of the preceding compositions comprising a whitening agent.
1.39 Any of the preceding compositions comprising a whitening agent selected from a whitening active selected from the group consisting of peroxides, metal chlorites, perborates, percarbonates, peroxyacids, hypochlorites, and combinations thereof.
1.40 The composition of 1.39, wherein the whitening agent is titanium dioxide.
1.41 Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate), or hydrogen peroxide polymer complexes such as hydrogen peroxide-polyvinyl pyrrolidone polymer complexes.
1.42 Any of the preceding compositions further comprising a polymer, e.g., an anionic polymer, for example a polycarboxylate polymer (e.g., PVM/MA copolymer, in an amount of from 0.1-5%, e.g., 0.2-2%, e.g., 0.3-1%.
1.43 Any of the preceding compositions further comprising microcrystalline cellulose and/or sodium carboxymethylcellulose, e.g., in an amount of from 0.1-5%, e.g., 0.5-2%, e.g. 1%.
1.44 Any of the preceding compositions further comprising one or both of:
   a. Polyethylene glycol in an amount of from 1-6% (e.g., 3% wt.); and
   b. Propylene glycol in an amount of from 1-6% (e.g., 4% wt.).
1.45 Any of the preceding compositions further comprising polyvinylpyrrolidone (PVP) in an amount of from 0.5-3 wt. %, e.g. about 1.25 wt. %.
1.46 Any of the preceding compositions further comprising an agent that interferes with or prevents bacterial attachment, e.g., ELA or chitosan.
1.47 Any of the preceding compositions, comprising glycerin in an amount from 20% - 45% by wt. of the composition (e.g., about 20% by wt., e.g., about 22% by wt., e.g., about 25% by wt, e.g., about 30%, e.g., about 35%, e.g., about 40%, e.g., about 43%)
1.48 Any of the preceding compositions wherein the humectant system comprises glycerin and sorbitol in a wt% ratio (glycerin:sorbitol) from 1:0.10 to 0.75:1, wherein the wt% is by weight of the humectant system.
1.49 Any of the preceding compositions, wherein the sorbitol is in an amount from 2.5% - 30 % by wt of the total composition (e.g., about 3.5%, about 7%, about 15%, about 17%, about 20%, about 24%, about 24.5%, about 25%, about 30%)
1.50 Any of compositions 1.0 -1.47, wherein the sorbitol is in an amount from 5% - 35% by wt of the total composition (e.g., about 5%, about 10%, about 15%, about 17%, about 20%, about 25%, about 30%).
1.51 Any of the preceding compositions, wherein the sorbitol is in an amount from 2.5% - 25% by wt. of the total composition (e.g., about 3%, e.g., about 3.5%, e.g., about 5%, e.g., about 17%, e.g., about 20%, e.g., about 21%, e.g., about 24%, e.g., about 25%).
1.52 Any of the preceding compositions, wherein the sorbitol is in an amount from 5% - 25% by wt. of the total composition.
1.53 Any of the preceding compositions, wherein the sorbitol is in an amount from 20% - 30% by wt. of the total composition.
1.54 Any of the preceding compositions, wherein the sorbitol is in an amount from 25% - 35% by wt. of the total composition.
1.55 Any of the preceding compositions comprising:
   a. Zinc Oxide about 1.0 wt.%
   b. Zinc Citrate about 0.5 wt%
   c. Stannous fluoride (e.g., about 0.45 wt%)
   d. Stannous pyrophosphate about 1.0 wt%
   e. A humectant system comprising glycerin and sorbitol, wherein the sorbitol is in an amount of about 3.5% by wt. of the total composition; and
   f. A thickening system comprising hydroxyethyl cellulose and carrageenan.
1.56 Any of 1.0 - 1.54, wherein the composition comprises:
   a. Zinc Oxide about 1.0 wt.%
   b. Zinc Citrate about 0.5 wt%
   c. Stannous fluoride (e.g., about 0.45 wt%)
   d. Stannous pyrophosphate about 1.0 wt%
   e. A humectant system comprising glycerin and sorbitol, wherein the sorbitol is in an amount of about 5% by wt. of the total composition; and
   f. A thickening system comprising hydroxyethyl cellulose and carrageenan.
1.57 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt%
   e. A humectant system comprising glycerin and sorbitol, wherein the sorbitol is in an amount of about 7% by wt. of the total composition; and
   f. A thickening system comprising hydroxyethyl cellulose and carrageenan.
1.58 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt%
   e. A humectant system comprising glycerin and sorbitol, wherein the sorbitol is in an amount of about 10 % by wt. of the total composition; and
   f. A thickening system comprising hydroxyethyl cellulose and carrageenan.
1.59 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 14% by wt. of the total composition; and
   f. A thickening system comprising hydroxyethyl cellulose and carrageenan.
1.60 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 15% by wt. of the total composition; and
   f. A thickening system comprising hydroxyethyl cellulose and carrageenan.
1.61 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 17% by wt. of the total composition; and
   f. A thickening system comprising hydroxyethyl cellulose and carrageenan.
1.62 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 20% by wt. of the total composition; and
   f. A thickening system comprising hydroxyethyl cellulose and carrageenan.
1.63 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 21 % by wt. of the total composition; and
   f. A thickening system comprising hydroxyethyl cellulose and carrageenan.
1.64 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 24.5 % by wt. of the total composition; and
   f. A thickening system comprising hydroxyethyl cellulose and carrageenan.
1.65 Any of 1.0 - 1.54, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 25 % by wt. of the total composition; and
   f. A thickening system comprising hydroxyethyl cellulose and carrageenan.
1.66 Any of 1.0 - 1.53, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt%;
   e. A humectant system comprising glycerin and sorbitol, wherein the sorbitol is in an amount of about 30 % by wt. of the total composition; and
   f. A thickening system comprising hydroxyethyl cellulose and carrageenan.
1.67 Any of 1.0 - 1.53, wherein the composition comprises:
   a. Zinc Oxide about 1.0 wt.%
   b. Stannous pyrophosphate about 1.0 wt%;
   c. Stannous fluoride (e.g., about 0.45 wt%); and
   d. A humectant system comprising glycerin and sorbitol, wherein the sorbitol is in an amount of about 35% by wt. of the total composition;
   e. Wherein zinc oxide is the only source of zinc in the composition; and
   f. A thickening system comprising hydroxyethyl cellulose and carrageenan.
1.68 Any of Composition 1.54-1.66 further comprising a citrate buffer system, wherein the buffer system comprises tri-sodium citrate and citric acid (e.g., the buffer system being about 2.1 wt% of the composition).
1.69 Any of the preceding compositions effective upon application to the oral cavity, e.g., by rinsing, optionally in conjunction with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit gingivitis, (vi) promote healing of sores or cuts in the mouth, (vii) reduce levels of acid producing bacteria, (viii) to increase relative levels of arginolytic bacteria, (ix) inhibit microbial biofilm formation in the oral cavity, (x) raise and/or maintain plaque pH at levels of at least pH 5.5 following sugar challenge, (xi) reduce plaque accumulation, (xii) treat, relieve or reduce dry mouth, (xiii) clean the teeth and oral cavity (xiv) reduce erosion, (xv) prevents stains and/or whiten teeth, (xvi) immunize the teeth against cariogenic bacteria; and/or (xvii) promote systemic health, including cardiovascular health, e.g., by reducing potential for systemic infection via the oral tissues.
1.70 Any of the preceding oral compositions, wherein the oral composition may be any of the following oral compositions selected from the group consisting of: a toothpaste or a dentifrice, a mouthwash or a mouth rinse, a topical oral gel, and a denture cleanser.
1.71 A composition obtained or obtainable by combining the ingredients as set forth in any of the preceding compositions.
1.72 Any preceding compositions, wherein zinc oxide and/or zinc citrate are the only sources of zinc.
1.73 Any of the preceding compositions, wherein stannous fluoride and stannous pyrophosphate are the only sources of stannous.
1.74 Any the preceding oral compositions, wherein the composition may be a dentifrice or a mouthwash.
1.75 Any the preceding oral compositions, wherein the composition may be any of the following selected from: a toothpaste, transparent paste, gel, mouth rinse, spray and chewing gum.
1.76 Any of the preceding oral compositions, wherein the composition is incorporated into a chewing gum.
1.77 A composition obtained or obtainable by combining the ingredients as set forth in any of the preceding compositions.
1.78 A composition for use as set for in any of the preceding compositions.

In another embodiment, the invention encompasses a method to improve oral health comprising applying an effective amount of the oral composition of any of the embodiments set forth above to the oral cavity of a subject in need thereof, e.g., a method to
i. reduce or inhibit formation of dental caries,
ii. reduce, repair or inhibit early enamel lesions, e.g., as detected by quantitative light- induced fluorescence (QLF) or electrical caries measurement(ECM),
iii. reduce or inhibit demineralization and promote remineralization of the teeth,
iv. reduce hypersensitivity of the teeth,
v. reduce or inhibit gingivitis,
vi. promote healing of sores or cuts in the mouth,
vii. reduce levels of acid producing bacteria,
viii. to increase relative levels of arginolytic bacteria,
ix. inhibit microbial bio film formation in the oral cavity,
x. raise and/or maintain plaque pH at levels of at least pH 5.5 following sugar challenge,
xi. reduce plaque accumulation,
xii. treat dry mouth,
xiii. enhance systemic health, including cardiovascular health,e.g., by reducing potential for systemic infection via the oral tissues,
xiv. Whiten teeth,
xv. reduce erosion of the teeth,
xvi.immunize (or protect) the teeth against cariogenic bacteria and their effects,
   and/or
xvii.clean the teeth and oral cavity.
The invention further comprises the use of sodium bicarbonate, sodium methyl cocoyl taurate (tauranol), MIT, and benzyl alcohol and combinations thereof in the manufacture of a Composition of the Invention, e.g., for use in any of the indications set forth in the above method of Composition 1.0, *et seq.*

### DETAILED DESCRIPTION

As used herein, the term "oral composition" means the total composition that is delivered to the oral surfaces. The composition is further defined as a product which, during the normal course of usage, is not, the purposes of systemic administration of particular therapeutic agents, intentionally swallowed but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition may be dual phase dispensed from a separated compartment dispenser.

### Stannous Ion Source

In some embodiments, the first stannous source comprises a stannous source selected from stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or mixtures thereof. In some embodiments, the first stannous source comprises stannous fluoride.

### Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al., each of which are incorporated herein by reference. Representative fluoride ion sources used with the present invention (e.g., Composition 1.0 *et seq*.) include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

### Surfactants

The invention may in some embodiments contain anionic surfactants, e.g., the Compositions of Composition 1.0, *et seq.,* for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocomo-glyceride sulfate; higher alkyl sulfates, such as sodium lauryl sulfate; higher alkyl-ether sulfates, e.g., of formula CH₃(CH₂)ₘCH₂(OCH₂CH₂)ₙOS0₃X, wherein m is 6-16, e.g., 10, n is 1-6, e.g., 2, 3 or 4, and X is Na or , for example sodium laureth-2 sulfate (CH₃(CH2)₁₀CH₂(OCH₂CH₂)₂OS0₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆-₃o alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., 1.5%.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al., herein incorporated by reference. Certain cationic surfactants can also act as germicides in the compositions.

Illustrative nonionic surfactants of Composition 1.0, *et seq.*, that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

In another embodiment illustrative zwitterionic surfactants of Composition 1.0, *et seq.,* that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

The surfactant or mixtures of compatible surfactants can be present in the compositions of the present invention in 0.1% to 5%, in another embodiment 0.3% to 3% and in another embodiment 0.5% to 2% by weight of the total composition.

### Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

### Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 0.1 wt. % pyrophosphate ions, e.g., 0.1 to 3 wt 5, e.g., 0.1 to 2 wt %, e.g., 0.1 to 1 wt%, e.g., 0.2 to 0.5 wt%. The pyrophosphates also contribute to preservation of the compositions by lowering water activity.

In various embodiments of the present disclosure (e.g., Composition 1.0 *et seq),* the compositions further comprise one or more anticalculus (tartar control) agents. Suitable anticalculus agents include without limitation mono-phosphates (e.g. monobasic, dibasic or tribasic phosphate) and P1-6 polyphosphates (e.g., pyrophosphates, tripolyphosphate, tetraphosphates and hexametaphosphate salts, zinc salts (e.g., zinc citrate, zinc chloride, zinc citrate trihydrate), Gantrez^{®} (a copolymer of methylvinyl ether (PVM) and maleic acid (MA)), polyaminopropanesulfonic acid (AMPS), polypeptides, polyolefin sulfonates, polyolefin phosphates, and diphosphonates. In certain embodiments, the other anticalculus agents are alkali and/or alkaline earth metal phosphate salts, for example, sodium, potassium or calcium salts. In certain embodiments, the composition includes mono-phosphates (e.g. monobasic, dibasic or tribasic phosphate), P1-6 polyphosphates, Gantrez, or a combination thereof. Still in certain embodiments, the composition includes sodium tripolyphosphate, tetrasodium pyrophosphate, Gantrez, or a combination thereof.

### Polymers

The oral care compositions of the invention also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1 :4 to 4: 1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1 : 1 copolymers of acrylic acid with methyl or hydroxyethyl methacrylate, methyl or ethyl acrylate, isobutyl vinyl ether or N-vinyl-2-pyrrolidone.

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid, incorporated herein by reference.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, xanthan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

### Abrasives

Natural calcium carbonate is found in rocks such as chalk, limestone, marble and travertine. It is also the principle component of egg shells and the shells of mollusks. The natural calcium carbonate abrasive of the invention is typically a finely ground limestone which may optionally be refined or partially refined to remove impurities. For use in the present invention, the material has an average particle size of less than 10 microns, e.g., 3-7 microns, e.g. about 5.5 microns. For example a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004% by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ^{®} 25-11 FG from GMZ.

Precipitated calcium carbonate is generally made by calcining limestone, to make calcium oxide (lime), which can then be converted back to calcium carbonate by reaction with carbon dioxide in water. Precipitated calcium carbonate has a different crystal structure from natural calcium carbonate. It is generally more friable and more porous, thus having lower abrasivity and higher water absorption. For use in the present invention, the particles are small, e.g., having an average particle size of 1 - 5 microns, and e.g., no more than 0.1 %, preferably no more than 0.05% by weight of particles which would not pass through a 325 mesh. The particles may for example have a D50 of 3-6 microns, for example 3.8=4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high water absorption, e.g., at least 25 g/100g, e.g. 30-70 g/100g. Examples of commercially available products suitable for use in the present invention include, for example, Carbolag^{®} 15 Plus from Lagos Industria Quimica.

In certain embodiments the invention may comprise additional calcium-containing abrasives, for example calcium phosphate abrasive, e.g., tricalcium phosphate (Ca₃(PO₄)₂), hydroxyapatite (Ca₁₀(PO₄)₆(OH)₂), or dicalcium phosphate dihydrate (CaHPO₄ · 2H₂0, also sometimes referred to herein as DiCal) or calcium pyrophosphate, and/or silica abrasives, sodium metaphosphate, potassium metaphosphate, aluminum silicate, calcined alumina, bentonite or other siliceous materials, or combinations thereof. Any silica suitable for oral care compositions may be used, such as precipitated silicas or silica gels. For example synthetic amorphous silica. Silica may also be available as a thickening agent, e.g., particle silica. For example, the silica can also be small particle silica (e.g., Sorbosil AC43 from PQ Corporation, Warrington, United Kingdom). However the additional abrasives are preferably not present in a type or amount so as to increase the RDA of the dentifrice to levels which could damage sensitive teeth, e.g., greater than 130.

### Water

Water is present in the oral compositions of the invention (e.g., Composition 1.0 *et seq*). Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

### Humectants

Within certain embodiments of the oral compositions (e.g. Composition 1.0 *et seq*), it is also desirable to incorporate a humectant to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerine, sorbitol, xylitol, propylene glycol as well as other polyols and mixtures of these humectants. Mixtures of glycerine and sorbitol may be used in certain embodiments as the humectant component of the compositions herein.

The present invention in its method aspect involves applying to the oral cavity a safe and effective amount of the compositions described herein.

The compositions and methods according to the invention (e.g., Composition 1.0 *et seq*) can be incorporated into oral compositions for the care of the mouth and teeth such as toothpastes, transparent pastes, gels, mouth rinses, sprays and chewing gum.

As used throughout, ranges are used as shorthand for describing each and every value that is within the range. Any value within the range can be selected as the terminus of the range. In addition, all references cited herein are hereby incorporated by reference in their entireties. In the event of a conflict in a definition in the present disclosure and that of a cited reference, the present disclosure controls. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention as many variations are possible without departing from the spirit and scope thereof. Various modifications of the invention in addition to those shown and described herein should be apparent to those skilled in the art and are intended to fall within the appended claims.

### Example 1

**Table 1.**

| **REPRESENTATIVE TOOTHPASTE FORMULATIONS** | | | |
|---|---|---|---|
| | **Formula A** | **Formula B** | **Formula C** |
| Material Description | Wt% | Wt% | Wt% |
| GLYCERIN - USP, EP VEG | 43.2 | 38.5 | 33.5 |
| ABRASIVE SILICA | 19.0 | 19.0 | 19.0 |
| SORBITOL - NON-CRYSTAL - 70% SOLN USP, EP | | 5.0 | 10.0 |
| DEMINERALIZED WATER | Q.S. | Q.S. | Q.S. |
| AMORPHOUS SILICA | 5.0 | 5.0 | 5.0 |
| HUMECTANT OTHER THAN GLYCERIN OR SORBITOL | 4.0 | 4.0 | 4.0 |
| POLYMERS | 4.25 | 4.0 | 4.0 |
| ALKALI PHOSPHATE SALTS | 3.0 | 3.0 | 3.0 |
| ANIONIC SURFACTANT | 1.75 | 1.75 | 1.75 |
| FLAVORS, COLORS, SWEETENERS | 2.25 | 2.25 | 2.25 |
| STANNOUS PYROPHOSPHATE | 1.0 | 1.0 | 1.0 |
| ZWITTERIONIC SURFACTANT | 1.0 | 1.0 | 1.0 |
| Microcrystalline Cellulose/Sodium CMC NF | 1.0 | 1.0 | 1.0 |
| ZINC OXIDE | 1.0 | 1.0 | 1.0 |
| COPOLYMER OF METHYLVINYL ETHER (PVM) AND MALEIC ACID (MA) | 0.606 | 0.606 | 0.606 |
| CITRATE BUFFER | 2.1 | 2.1 | 2.1 |
| 85% SYRUPY PHOSPHORIC ACID - FOOD GRADE | 0.55 | 0.55 | 0.55 |
| ZINC CITRATE TRIHYDRATE | 0.5 | 0.5 | 0.5 |
| STANNOUS FLUORIDE, USP | 0.454 | 0.454 | 0.454 |
| HYDROXYETHYLCELLULOSE | 0.3 | 0.25 | 0.25 |
| CARRAGEENAN CONCENTRATE | 0.3 | 0.25 | 0.25 |
| Total Components | 100.0 | 100.0 | 100.0 |
| Total Water | ~ 9.3 | 10.8 | 12.3 |

**Table 1. (continued)**

| | **Formula D** | **Formula E** | **Formula F** | **Formula G** |
|---|---|---|---|---|
| Material Description | Wt% | Wt% | Wt% | Wt% |
| GLYCERIN - USP, EP VEG | 28.5 | 23.5 | 22.5 | 22.6 |
| ABRASIVE SILICA | 19.0 | 19.0 | 20.0 | 20.0 |
| SORBITOL - NON-CRYSTAL - 70% SOLN USP, EP | 15.0 | 20.0 | 30.0 | 30.0 |
| DEMINERALIZED WATER | Q.S. | Q.S. | Q.S. | Q.S. |
| AMORPHOUS SILICA | 5.0 | 5.0 | | |
| HUMECTANT OTHER THAN GLYCERIN OR SORBITOL | 4.0 | 4.0 | | |
| POLYMERS | 4.0 | 4.0 | 4.0 | 4.0 |
| ALKALI PHOSPHATE SALTS | 3.0 | 3.0 | 3.0 | 3.0 |
| ANIONIC SURFACTANT | 1.75 | 1.75 | 1.20 | 1.20 |
| FLAVORS, COLORS, SWEETENERS | 2.25 | 2..25 | 1.45 | 1.45 |
| STANNOUS PYROPHOSPHATE | 1.0 | 1.0 | 1.000 | 1.0 |
| ZWITTERIONIC SURFACTANT | 1.0 | 1.0 | 0.60 | 0.60 |
| Microcrystalline Cellulose/Sodium CMC NF | 1.0 | 1.0 | 1.0 | 1.0 |
| ZINC OXIDE | 1.0 | 1.0 | 1.0 | 1.0 |
| COPOLYMER OF METHYLVINYL ETHER (PVM) AND MALEIC ACID (MA) | 0.606 | 0.606 | | |
| CITRATE BUFFER | 2.1 | 2.1 | 2.1 | 2.1 |
| 85% SYRUPY PHOSPHORIC ACID - FOOD GRADE | 0.55 | 0.55 | 0.55 | 0.55 |
| ZINC CITRATE TRIHYDRATE | 0.5 | 0.5 | 0.5 | |
| STANNOUS FLUORIDE, USP | 0.454 | 0.454 | 0.454 | 0.454 |
| HYDROXYETHYLCELLULOSE | 0.25 | 0.25 | 0.3 | 0.3 |
| CARRAGEENAN CONCENTRATE | 0.25 | 0.25 | 0.3 | 0.3 |
| THICKENING SILICA | | | 1.25 | 1.50 |
| Total Components | 100.0 | 100.0 | 100.0 | 100.0 |
| Total Water | 13.8 | 15.3 | 18.1 | 18.3 |

### Example 2

*In vitro* assay for monitoring metal ion deposition

**Table 2.**

| | | **A** | **B** | **C** | **D** | **E** | **F** | **G** |
|---|---|---|---|---|---|---|---|---|
| pH (10% Soln) | Initial | 7.10 | 6.87 | 6.77 | 6.86 | 7.28 | 6.96 | 7.18 |
| | 4wks-40C/75%RH | 6.73 | 7.16 | 7.01 | 6.66 | 7.26 | 6.99 | 7.27 |
| | 8wks-40C/75%RH | 6.89 | 7.12 | 7.17 | 7.16 | 7.22 | 7.09 | 7.22 |
| | 13wks-30C/65%RH | 6.94 | 7.22 | 7.08 | 6.95 | 7.10 | 7.10 | 7.29 |
| | 13wks-40C/75%RH | 6.95 | 7.23 | 7.07 | 7.10 | 7.14 | 7.16 | 7.33 |
| | | | | | | | | |
| Sol. Fluoride (ppm) | Initial | 1128 | 1036 | 1042 | 1065 | 1075 | 1088 | 1048 |
| | 4wks-40C/75%RH | 950 | 931 | 901 | 889 | 896 | 1002 | 916 |
| | 8wks-40C/75%RH | 869 | 865 | 1019 | 803 | 838 | 987 | 867 |
| | 13wks-30C/65%RH | 100 9 | 764 | 899 | 840 | 910 | 893 | 876 |
| | 13wks-40C/75%RH | 791 | 692 | 713 | 688 | 719 | 705 | 711 |
| | | | | | | | | |
| Total Tin (%) | Initial | 0.80 | 0.83 | 0.83 | 0.86 | 0.90 | 0.83 | 0.81 |
| | | | | | | | | |
| Sol. Tin (%) | Initial | 0.43 | 0.54 | 0.45 | 0.60 | 0.55 | 0.64 | 0.45 |
| | 4wks-40C/75%RH | 0.40 | 0.47 | 0.55 | 0.52 | 0.53 | 0.65 | 0.49 |
| | 8wks-40C/75%RH | 0.39 | 0.43 | 0.47 | 0.50 | 0.50 | 0.64 | 0.48 |
| | 13wks-30C/65%RH | 0.43 | 0.47 | 0.43 | 0.58 | 0.52 | 0.66 | 0.42 |
| | 13wks-40C/75%RH | 0.38 | 0.41 | 0.46 | 0.51 | 0.39 | 0.59 | 0.40 |
| | | | | | | | | |
| Total Zinc (%) | Initial | 0.92 | 0.97 | 0.96 | 0.95 | 0.89 | 1.00 | 0.72 |
| | | | | | | | | |
| Sol. Zinc (%) | Initial | 0.65 | 0.60 | 0.59 | 0.59 | 0.69 | 0.62 | 0.47 |
| | 4wks-40C/75%RH | 0.67 | 0.54 | 0.56 | 0.53 | 0.61 | 0.59 | 0.43 |
| | 8wks-40C/75%RH | 0.52 | 0.50 | 0.50 | 0.53 | 0.49 | 0.57 | 0.43 |
| | 13wks-30C/65%RH | 0.32 | 0.55 | 0.41 | 0.50 | 0.61 | 0.61 | 0.48 |
| | 13wks-40C/75%RH | 0.28 | 0.46 | 0.50 | 0.40 | 0.51 | 0.54 | 0.33 |
| | | | | | | | | |

Table 2 represents Stability summary of stannous fluoride toothpastes (described in Table 1).

Formulations in this invention can utilize up to 30% sorbitol to provide processing flexibility and, in some embodiments, may contain only a single zinc source. These formulas can contain up to about 18% total water due to contributions of sorbitol and other raw materials with some water content.

Formulas of Table 1 in Example 1 are evaluated for chemical and physical stability per ICH accelerated aging/stress guidelines and compared to a positive control formulation (Formula A) which does not contain any sorbitol. Based upon the data in Table 2, Formulas B-G of Table 1 are sufficiently stable for fluoride, soluble tin and soluble zinc and are acceptably buffered to maintain pH within 6.5-7.5 target range - See, Table 2.

The intention of these formulas is to provide a source of both soluble and insoluble metals within the toothpaste. Without being bound by theory, the insoluble sources may act as a reservoir that remain stable during shelf-life of the toothpaste but become available on dilution with saliva during brushing. The stability data of Table 2 demonstrates success in this practice, throughout the accelerated aging of the formulas, as both total tin and zinc are considerably greater percentages compared to soluble components. Also, the soluble components show relatively little change over the 13-week accelerated aging period which indicates successful metal stability, showing that compositions with some amount of sorbitol worked at parity to those compositions with only glycerin.

### Example 3

The University of Manchester anaerobic model is to provide a more sensitive indication of potential efficacy of the formulas described herein. In this model, saliva collected from 4 healthy volunteers is pooled together and used as inoculum. Each sample is treated in triplicate twice a day for 8 days. Biofilm is recovered after 16 treatments to measure for ATP (RLU) as an end point for viable bacteria. Toothpastes demonstrating lower ATP scores provide more effective antibacterial performance. Market based toothpaste formulations containing NaF and KNO3 actives are used as the "Negative Control" referred to in the tables below.

There are two separate tests in this study conducted with toothpaste prototypes of this invention. Formulas referred to here are the same as those described in Table 1, of Example 1, above.

In the first test, it is surprisingly found that both Formula B (containing 5% sorbitol, zinc citrate and zinc oxide) and Formula G (containing 30% sorbitol, only ZnO) perform statistically significantly better than the Formula A standard with no sorbitol. As can be seen, Formula G (30% sorbitol, single ZnO) provided superior performance over all other samples.

**Table 3. Viable Bacteria as ATP (RLU) - Manchester Model, Test 1**

| **Sample** | **Avg Log RLU** | **Statistical Comparison*** |
|---|---|---|
| Negative Control** | 4.652 +/- 0.213 | A |
| Formula A | 3.748 +/- 0.146 | B |
| Formula B | 3.370 +/- 0.120 | C |
| Formula G | 3.097 +/- 0.182 | D |

| | | |
|---|---|---|
| * Means that don't share common letter=Sign. Diff @95% CI, Tukey method, N=26 per cell ** Negative Control is the market-based formula containing NaF and KNO3 as the active ingredients. | | |

Both Formula A standard (Positive Control) and Formula G are included in an additional study and compared to Formula C (10% sorbitol, zinc oxide and zinc citrate). Formula G containing 30% sorbitol and zinc oxide (where zinc oxide is the only zinc source) performed very well, and at parity with the Positive Control (Formula) that has two zinc sources: zinc oxide and zinc citrate. All tested stannous fluoride formulations (Formulations A-G) performed significantly better than the negative control at controlling anaerobic biofilm.

**Table 4. Viable Bacteria as ATP (RLU) - Manchester Model, Test 2**

| **Sample** | **Avg Log RLU** | **Statistical Comparison*** |
|---|---|---|
| Negative Control** | 4.771 +/- | A |
| | 0.130 | |
| Formula A | 3.781 +/- | C,D |
| | 0.122 | |
| Formula C | 4.127 +/- | B |
| | 0.177 | |
| Formula G | 3.698 +/- | D |
| | 0.153 | |

| | | |
|---|---|---|
| * Means that don't share common letter=Sign. Diff @95% CI, Tukey method, N=26 per cell ** Negative Control is a market-based formula containing NaF and KNO3 as the active ingredients | | |

**Plaque glycolysis Model:** Formulas referred to here are the same as those described in Table 1 above.

An in vitro adaptation of a Glycolysis Model described by White, et. al., Journal of Clinical Dentistry, #6 Special Issue, p 69-78, (1995), the contents of which are incorporated herein by reference, is used to indirectly measure biofilm health. Briefly, the method quantifies the glycolytic effects of toothpaste formulas on treated in vitro biofilm pool of both anaerobic and aerobic bacteria. The efficacy of each toothpaste formula is based on biofilm pH change. A lower average pH change indicates reduction of viable bacteria and greater antibacterial performance of the respective test toothpaste. Finally, in these studies, an untreated cell is used as the negative control.

There are two separate plaque glycolysis tests conducted with toothpaste prototypes of this invention. In the first test, Formula A, Positive Control, is compared to Formula E (20% sorbitol, zinc citrate and zinc oxide). It is surprisingly found that the Formula E prototype is statically superior to the standard.

**Table 5. Plaque Glycolysis Study - Average pH Change with Treatment, Test 1**

| **Sample** | **Avg pH Change** | **Statistical Comparison*** |
|---|---|---|
| Untreated** | 2.425 +/- 0.095 | A |
| Formula A | 0.761 +/- 0.023 | B |
| Formula E | 0.567 +/- 0.018 | C |

| | | |
|---|---|---|
| * Means that don't share common letter=Sign. Diff @95% CI, Tukey method, N=3 per cell ** Negative Control, untreated biofilm | | |

A second plaque glycolysis study compares Formula C (10% sorbitol, zinc citrate and zinc oxide) and Formula F (30% sorbitol, zinc oxide and zinc citrate) to the Positive Control (Formula A) and Negative Control (untreated biofilm). The 10% sorbitol formula of Formula C is comparable to the no sorbitol standard (Formula A). Whereas, the 30% sorbitol formula (i.e, Formula F) performs significantly better than the Formula A Positive Control standard.

**Table 6. Plaque Glycolysis Study - Average pH Change with Treatment, Test 2**

| **Sample** | **Avg pH Change** | **Statistical Comparison*** |
|---|---|---|
| Untreated** | 2.633 +/- 0.102 | A |
| Formula A | 0.856 +/- 0.069 | B |
| Formula C | 1.009 +/- 0.098 | B |
| Formula F | 0.549 +/- 0.045 | C |

| | | |
|---|---|---|
| * Means that don't share common letter=Sign. Diff @95% CI, Tukey method, N=3 per cell ** Negative Control, untreated biofilm | | |

### Example 4

The addition of sorbitol surprisingly helps in the manufacturing of the oral care compositions of the present invention.

In one aspect, the addition of sorbitol allows lowering the temperature at certain steps of the manufacturing process and to reduce the time taken to hydrate and mix the gums - e.g., HEC and Carrageenan - present in the formulation. In one aspect the addition of sorbitol enables lowering the temperature, and the time, to heat and hydrate the gums from 80 degrees Celsius (without sorbitol) to 50 degrees Celsius (with the addition of sorbitol).

Furthermore, Table 7 and Table 8 (below) also show that the physical stability of certain formulations containing 10% sorbitol, for example, function at parity, and/or demonstrate a slight improvement than formulations without sorbitol. The Formulas referred to in Tables 7 and 8 are the same as detailed in Table 1 (Example 1) above. Therefore, the addition of sorbitol in certain formulations can aid in manufacturing efficiency while retaining the physical stability of formulations without sorbitol:

**Table 7.**

| **Formula A:** | | | | | | |
|---|---|---|---|---|---|---|
| **1.0%SnPyro, 0.3%HEC, 0.3%Carr** | | | | | | |
| | **4wk-25C** | **4wk-40C** | **8wk-25C** | **8wk-40C** | **13wk-25C** | **13wk-40C** |
| Shear Stress (Pa) | 177 | 179 | 173 | 169 | 165 | 179 |
| Static Yield (Pa) | 56.9 | 70.2 | 65.1 | 87.2 | 68.9 | 68.2 |
| Dynamic Yield (Pa) | 15 | 15 | 15 | 15 | 14.2 | 14.5 |
| Viscosity @ 1 RPM (cP) | 332,360 | 312,393 | 291,781 | 356,836 | 289,205 | 356,836 |

**Table 8.**

| **Formula C: 1.0%SnPyro, 0.25%HEC 0.25%Carr, 10%Sorbitol** | | | | | | |
|---|---|---|---|---|---|---|
| | 4wk-25C | 4wk-40C | 8wk-25C | 8wk-40C | 13wk-25C | 13wk-40C |
| Shear Stress (Pa) | 180.72 | 190.10 | 201.87 | 194.18 | 265.315 | 344.14 |
| Static Yield (Pa) | 55.10 | 65.1 | 75.70 | 56.10 | 93.4 | 111.0 |
| Dynamic Yield (Pa) | 23.40 | 17.40 | 9.01 | 11.0 | 14.70 | 11.50 |
| Viscosity @ 1 RPM (cP) | 435,386 | 363,259 | 337,507 | 267,943 | 418,664 | 352,568 |

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

While the present invention has been described with reference to embodiments, it will be understood by those skilled in the art that various modifications and variations may be made therein without departing from the scope of the present invention as defined by the appended claims.
The invention also refers to the following numbered embodiments, wherein the term "claim" refers to "embodiment".
1. An oral care composition comprising:
   a. A zinc source selected from the group consisting of: zinc oxide, zinc citrate, zinc lactate, and combinations thereof;
   b. a first source of stannous;
   c. a second source of stannous, wherein the second source of stannous comprises stannous pyrophosphate;
   d. a humectant system comprising glycerin and sorbitol, and wherein the sorbitol is in an amount from 2.5% - 35% by wt. of the total composition; and
   e. a thickening system comprising a thickening agent selected from the group consisting of carboxyvinyl polymers, carrageenan, hydroxyethyl cellulose, water-soluble salts of cellulose ethers, and combinations thereof.
2. The oral care composition of claim 1, wherein the zinc source comprises zinc oxide.
3. The oral care composition of claim 1, wherein the zinc source comprises zinc oxide and zinc citrate.
4. The oral care composition of any preceding claims, wherein the ratio of the amount of zinc oxide to zinc citrate is from 1.5:1 to 4.5:1.
5. The oral care composition of any preceding claims, comprising zinc citrate and zinc oxide, wherein the zinc citrate is present in an amount of from 0.25 to 1.0 wt%, and the zinc oxide may be present in an amount of from 0.75 to 1.25 wt%, based on the total weight of the composition.
6. The oral care composition of any preceding claims, wherein the first stannous ion source is stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or a mixture thereof.
7. The oral care composition of any preceding claims, wherein the first stannous ion source is stannous fluoride.
8. The oral care composition of any preceding claims, wherein the composition comprises one or more alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogen orthophosphate, monosodium phosphate, pentapotassium triphosphate and mixtures thereof.
9. The oral care composition of claim 8, wherein the composition comprises an alkali phosphate salt in an amount of from 1-20% by weight, based on the total weight of the composition.
10. Any of the preceding claims wherein the humectant system comprises glycerin and sorbitol in a wt% ratio (glycerin:sorbitol) from 1:0.10 to 0.75:1, by weight of the humectant system.
11. Any of the preceding compositions, wherein the sorbitol is in an amount from 2.5% - 25% by wt. of the total composition
12. Any of claims 1-10, wherein the sorbitol is in an amount from 5% - 35% by wt of the total composition.
13. The composition of claim 11, wherein the sorbitol is in an amount from 5% - 25% by wt of the total composition.
14. The composition of claim 11, wherein the sorbitol is in an amount from 25% - 35% by wt of the total composition.
15. A composition of any of the preceding claims, wherein the composition comprises:
   a. Zinc Oxide about 1.0 wt.%
   b. Zinc Citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 3.5% by wt. of the total composition.
16. A composition of any of claims 1-14, wherein the composition comprises:
   a. Zinc Oxide about 1.0 wt.%
   b. Zinc Citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 5% by wt. of the total composition.
17. A composition of any of claims 1-14, wherein the composition comprises:
   a. Zinc Oxide about 1.0 wt.%
   b. Zinc citrate about 0.5 wt%
   c. Stannous fluoride (e.g., about 0.45 wt%)
   d. Stannous pyrophosphate about 1.0 wt% and
   e. A humectant system comprising glycerin and sorbitol, wherein the sorbitol is in an amount of about 21 % by wt. of the total composition; and
   f. A thickening system comprising hydroxyethyl cellulose and carrageenan.
18. A composition of any of claims 1-14, wherein the composition comprises:
   a. Zinc Oxide about 1.0 wt.%
   b. Stannous pyrophosphate about 1.0 wt%;
   c. Stannous fluoride (e.g., about 0.45 wt%); and
   d. A humectant system comprising glycerin and sorbitol, wherein the sorbitol is in an amount of about 30 % by wt. of the total composition.
19. The composition of either claim 15 or 16, further comprising a citrate buffer system, wherein the buffer system comprises tri-sodium citrate and citric acid.
20. The composition of any of the preceding claims, wherein the composition may be any of the following selected from: a toothpaste, transparent paste, gel, mouth rinse, spray and chewing gum.
21. A method to improve oral health comprising applying an effective amount of the oral composition of any of the preceding claims, wherein the oral care composition is applied to the oral cavity of a subject in need thereof.

## Claims

1. An oral care composition comprising:
a. a zinc source;
b. a first source of stannous;
c. a second source of stannous, wherein the second source of stannous comprises stannous pyrophosphate;
d. a humectant system comprising glycerin and sorbitol; and
e. a thickening system comprising a thickening agent selected from the group consisting of carrageenan, hydroxyethyl cellulose, and water-soluble salts of cellulose ethers, and combinations thereof,
wherein the wt.% is based on the total weight of the oral care composition.

2. The oral care composition according to claim 1, wherein the zinc source is selected from the group consisting of: zinc oxide, zinc citrate, zinc lactate, and combinations thereof.

3. The oral care composition according to claim 1 or 2, wherein the zinc source comprises zinc oxide and zinc citrate.

4. The oral care composition according to claim 3, wherein the ratio of the amount of zinc oxide to zinc citrate is from 1.5:1 to 4.5:1.

5. The oral care composition according to any preceding claim, comprising zinc citrate and zinc oxide, wherein the zinc citrate is present in an amount of from 0.25 to 1.0 wt.%, and the zinc oxide may be present in an amount of from 0.75 to 1.25 wt.%, based on the total weight of the composition.

6. The oral care composition according to any preceding claim, wherein the first source of stannous is stannous fluoride; stannous chloride; stannous pyrophosphate; organic stannous carboxylate salts such as stannous formate, acetate, gluconate, lactate, tartrate, oxalate, malonate and citrate, stannous ethylene glyoxide, or a mixture thereof.

7. The oral care composition according to any preceding claim, wherein the first source of stannous is stannous fluoride.

8. The oral care composition according to any preceding claim, further comprising one or more alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, disodium hydrogen orthophosphate, monosodium phosphate, pentapotassium triphosphate and mixtures thereof; preferably wherein the composition comprises an alkali phosphate salt in an amount of from1 to 20 wt.%, based on the total weight of the composition.

9. The oral care composition according to any preceding claim, wherein the humectant system comprises glycerin and sorbitol in a wt.% ratio (glycerin:sorbitol) from 1:0.10 to 0.75:1, based on the weight of the humectant system.

10. The oral care composition according to any preceding claim, wherein the sorbitol is present in an amount of from 2.5 to 35 wt.% or from 2.5 to 25 wt.%, based on the total weight of the composition.

11. The oral care composition according to any one of claims 1 to 8, wherein the sorbitol is present in an amount of from 5 to 35 wt.%, preferably in an amount of from 5 to 25 wt.%; or in an amount of from 25 to 35 wt.%, based on the total weight of the composition.

12. A composition according to any preceding claim, wherein the composition comprises:
a. zinc oxide in an amount of about 1.0 wt.%;
b. zinc citrate in an amount of about 0.5 wt.%;
c. stannous fluoride in an amount of preferably about 0.45 wt.%;
d. stannous pyrophosphate in an amount of about 1.0 wt.%; and
e. a humectant system comprising glycerin and sorbitol, wherein the sorbitol is present in an amount of about 3.5 wt.%,
or wherein the composition comprises:
f. zinc oxide in an amount of about 1.0 wt.%;
g. zinc citrate in an amount of about 0.5 wt.%;
h. stannous fluoride in an amount of preferably about 0.45 wt.%;
i. stannous pyrophosphate in an amount of about 1.0 wt.%; and
j. a humectant system comprising glycerin and sorbitol, wherein the sorbitol is present in an amount of about 5 wt.%,
or wherein the composition comprises:
k. zinc oxide in an amount of about 1.0 wt.%;
l. zinc citrate in an amount of about 0.5 wt.%;
m. stannous fluoride in an amount of preferably about 0.45 wt.%;
n. stannous pyrophosphate in an amount of about 1.0 wt.%; and
o. a humectant system comprising glycerin and sorbitol, wherein the sorbitol is present in an amount of about 21 wt.%; and
p. a thickening system comprising hydroxyethyl cellulose and carrageenan,
or wherein the composition comprises:
q. zinc oxide in an amount of about 1.0 wt.%;
r. stannous pyrophosphate in an amount of about 1.0 wt.%;
s. stannous fluoride in an amount of preferably about 0.45 wt.%; and
t. a humectant system comprising glycerin and sorbitol, wherein the sorbitol is present in an amount of about 30 wt.%,
wherein the wt.% is based on the total weight of the composition.

13. The composition according to claim 10, further comprising a citrate buffer system, wherein the buffer system comprises tri-sodium citrate and citric acid.

14. The composition according to any preceding claim, wherein the composition is in a form selected from: a toothpaste, transparent paste, gel, mouth rinse, spray and chewing gum.

15. A composition according to any preceding claim for use in a method to improve oral health comprising applying an effective amount of said oral composition, wherein the oral care composition is applied to the oral cavity of a subject in need thereof.
